(19) Europäisches Patentamt
European Patent Office
Office européen des brevets

(11) Veröffentlichungsnummer: **0 133 912**
**B1**

(12) **EUROPÄISCHE PATENTSCHRIFT**

(45) Veröffentlichungstag der Patentschrift: **04.11.87**

(51) Int. Cl.⁴: **C 07 C 19/02**, C 07 C 17/02

(21) Anmeldenummer: **84107638.3**

(22) Anmeldetag: **02.07.84**

(54) **Verfahren zur Herstellung von 1,1,2-Trichlor-2-methyl-propan.**

(30) Priorität: **25.08.83 DE 3330609**
**25.04.84 DE 3415334**

(43) Veröffentlichungstag der Anmeldung: **13.03.85 Patentblatt 85/11**

(45) Bekanntmachung des Hinweises auf die Patenterteilung: **04.11.87 Patentblatt 87/45**

(84) Benannte Vertragsstaaten: **AT BE CH DE FR GB IT LI NL**

(56) Entgegenhaltungen:
**BEILSTEINS HANDBUCH DER ORGANISCHEN CHEMIE, Band 1, 4. Auflage, Julius Springer Verlag, 1918, Seite 126, Zeilen 29-33, Berlin; & CHEMISCHES ZENTRALBLATT 1905, 76, 667-668**
**CHEMISCHES ZENTRALBLATT, Band 110, Nr. 25, 1939, Seiten 4221-4223, Berlin; D.W. TISCHTSCHENKO "Untersuchungen auf dem Gebiet der Chlorderivate der Fettreihe. XIV. Die additive Fähigkeit der Doppelbindung am quaternären Kohlenstoff", & CHEM. J. SER. A, J. ALLG. CHEM. Band 8, 1232-1246**
**JOURNAL OF THE AMERICAN CHEMICAL SOCIETY, Band 68, Nr. 5, 11. Mai 1946, Seiten 785-789, Columbus, Ohio, US; A. MOORADIAN et al.: "4-Chloro-3-methyl-3-butenonitrile and related compounds"**

(73) Patentinhaber: **HÜLS AKTIENGESELLSCHAFT, - RSP Patente / PB 15 - Postfach 13 20, D-4370 Marl 1 (DE)**

(72) Erfinder: **Müller, Dieter Jürgen, Dr., Stargarder Strasse 30, D-4370 Marl (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 133 912 B1

LIBER, STOCKHOLM 1987

**Beschreibung**

Die Erfindung betrifft ein Verfahren zur Herstellung von 1,1,2-Trichlor-2-methyl-propan durch Umsetzung von 1-Chlor-2-methyl-propen mit Sulfurylchlorid, vorzugsweise unter Einwirkung von Licht, insbesondere UV-Licht und/oder in Gegenwart von Aldehyden als Katalysator.

Es ist bisher kein Verfahren bekannt, das es gestattet, 1,1,2-Trichlor-2-methyl-propan selektiv aus $C_4$-Verbindungen herzustellen.

Bei der direkten Chlorierung von Isobuten fällt es in geringem Umfang neben den Hauptprodukten 3-Chlor-2-methylpropen und 1-Chlor-2-methyl-propen mit weiteren Tri-, Tetra- und Pentachlorderivaten als höher chloriertes Nebenprodukt schwer isolierbar an.

Ebenso liefert die photochemische Chlorierung von Isobutan in der Gasphase das 1,1,2-Trichlor-2-methyl-propan nur als Nebenprodukt (Beilstein E III 1, S. 320 (1958): Ing. eng. Chem. 40 (1945) 1488).

Die Weiterchlorierung bereits partiell chlorierter $C_4$-Verbindungen verläuft ebenfalls wenig selektiv.

So liefert die Chlorierung von 2-Chlor- und 1,2-Dichlor-2-methyl-propan in Flüssigphase 1,1,2-Trichlor-2-methylpropan nur als Nebenprodukt (Beilstein E III 1, S. 320 (1958): Am. Soc. 58 (1936) 1028, 1029), auch wenn in Gegenwart von $AlCl_3$ gearbeitet wird (Beilstein E IV 1, S. 293 (1972): Bl. chem. Soc. Japan 30 (1957) 218, 220).

Die Chlorierung von 1-Chlor-2-methyl-propen in Gegenwart von Natriumhydrogencarbonat bei 0°C liefert neben 68 % 3,3-Dichlor-2-methyl-propen nur 32 % 1,1,2-Trichlor-2-methyl-propan (Chem. Abstr. 33 (1939) 4190 bzw. Chemisches Zentralblatt 110 (1939) Nr. 25, Seiten 4221 bis 4223).

Aus dem Journal of the American Chemical 68 (1946) Nr. 5, Seiten 785 bis 789, war es bekannt, das isomere 3-Chlor-2-methyl-propen mit Sulfurylchlorid zum 1,2,3-Trichlor-2-methyl-propan umzusetzen. Die analoge Reaktion von 1-Chlor-2-methyl-propen führt jedoch neben der gewünschten Bildung von 1,1,2-Trichlor-2-methyl-propan zur Bildung der unerwünschten Nebenprodukte 3,3-Dichlor-2-methyl-propen und 1,3-Dichlor-2-methyl-propen.

Allen bekannten Verfahren ist gemeinsam, daß das gewünschte 1,1,2-Trichlor-2-methyl-propan nur als Nebenprodukt anfällt.

Der Erfindung liegt die Aufgabe zugrunde, ein Verfahren bereitzustellen, das es gestattet, 1,1,2-Trichlor-2-methyl-propan als Hauptprodukt in technisch einfacher Weise herzustellen.

Diese Aufgabe wird erfindungsgemäß dadurch gelöst, daß man 1-Chlor-2-methyl-propen mit Sulfurychlorid unter Einwirkung von Licht, insbesondere von UV-Licht und/oder in Gegenwart von Aldehyden als Katalysator gemäß folgender Gleichung, bevorzugt in der Flüssigphase im Temperaturbereich zwischen 30 und 65°C, umsetzt.

$$Cl-CH=\overset{\overset{CH_3}{|}}{C}-CH_3 + SO_2Cl_2 \xrightarrow[\text{Aldehyde}]{h.v.} Cl_2CH-\underset{\underset{Cl}{|}}{\overset{\overset{CH_3}{|}}{C}}-CH_3 + SO_2$$

Die Umsetzung von Sulfurychlorid mit dem isomeren 3-Chlor-2-methyl-propen ergibt in bekannter Additionsreaktion 1,2,3-Trichlor-2-methyl-propan mit zufriedenstellender Ausbeute von 83 % (J. Am. Chem. Soc. 68 (1946) 787).

Die analoge Reaktion von Sulfurychlorid mit 1-Chlor-2-methyl-propen führt überraschenderweise zu keinem eindeutigen Ergebnis. Die Reaktion, die an der Entwicklung von gasförmigem $SO_2$ verfolgt werden kann, verläuft einerseits sehr langsam, andererseits ist das Produktspektrum wenig reproduzierbar. Neben dem gewünschten 1,1,2-Trichlor-2-methyl-propan entstehen insbesondere in unterschiedlichen Mengen auch 3,3-Dichlor-2-methyl-propen und 1,3-Dichlor-2-methyl-propen.

Es wurde gefunden, daß die unbefriedigende Repruduzierbarkeit des Produktspektrums auf geringe Spuren verschiedener Stabilisatoren im ppm-Bereich in 1-Chlor-2-methyl-propen zurückzuführen ist. Es hat sich dabei herausgestellt, daß einige gebräuchliche Stabilisatoren für Chlorkohlenwasserstoffe die Reaktion sowohl hinsichtlich der Geschwindigkeit als auch des Produktspektrums beeinflussen und katalysierende oder inhibierende Wirkung zeigen, wobei sich bei Stabllisatorabmischungen die Effekte kompensieren oder verstärken können. Insbesondere ist die Anwesenheit von Stickstoffbasen nachteilig, weil diese den Reaktionsablauf in Richtung Substitution verschieben. Für die erfindungsgemäße Umsetzung eignet sich ein 1-Chlor-2-methyl-propen, das weniger als 1 ppm eines Stabilisators, insbesondere einer Stickstoffbase, enthält.

Bei Einsatz von einem solchen, weitgehend stabilisatorfreien 1-Chlor-2-methyl-propen hat sich gezeigt, daß die Einwirkung von Licht, insbesondere von UV-Licht, die Reaktion sowohl beschleunigt als auch praktisch quantitativ in Richtung Additionsprodukt verschiebt.

Als UV-Licht eignet sich insbesondere Licht einer Wellenlänge von 200 bis 400 nm.

Anstelle von Licht kann man die Umsetzung auch in Gegenwart von Aldehyden als Katalysator, vorzugsweise von aliphatischen bzw. olefinischen Aldehyden, wie beispielsweise von Isobutanal, durchführen. Dieser Aldehyd hat den Vorteil, nicht systemfremd zu sein, da er bereits in Spuren in 1-Chlor-2-methyl-propen infolge hydrolytischer und/oder oxidativer Vorgänge vorhanden sein kann.

Es wurde gefunden, daß bereits 10 bis 100 ppm der Aldehyde katalytisch wirksam sind und die Reaktion in

Richtung Addition verstärken. Die Reaktionsgeschwindigkeit ist allerdings geringer als unter der Einwirkung von UV-Licht.

Im allgemeinen verwendet man 10 bis 10 000 ppm an Aldehyden, vorzugsweise werden etwa 100 ppm bis 1 000 ppm an Aldehyden zugegeben, obwohl auch höhere Gehalte bis 10 000 ppm und mehr möglich sind. Damit können für die Umsetzung von 1-Chlor-2-methyl-propen zu 1,1,2-Trichlor-2-methyl-propan Selektivitäten bis zu 96 % erreicht werden. Man kann die Umsetzung auch unter der Einwirkung von Licht und in Gegenwart von Aldehyden als Katalysator durchführen.

Damit ist es erstmals möglich, 1,1,2-Trichlor-2-methyl-propan mit einer Selektivität von über 99 % aus 1-Chlor-2-methyl-propen herzustellen. Das Verfahren eignet sich sowohl für einen diskontinuierlichen Prozeß beispielsweise in einem Rührreaktor als auch für einen kontinuierlichen Prozeß beispielsweise in einem Rohrreaktor oder einer Kaskade.

Die Reaktion führt man bevorzugt in der Flüssigphase durch. Das Sulfurylchlorid kann man, bezogen auf 1-Chlor-2-methyl-propen, in stöchiometrischer Menge einsetzen. Bevorzugt setzt man es in stöchiometrischem UUnterschuß ein.

Bei der weiteren Ausgestaltung des Verfahrens hat es sich als zweckmäßig erwiesen, Sulfurylchlorid im stöchiometrischen Unterschuß einzusetzen, da sich nicht umgesetztes überschüssiges 1-Chlor-2-methyl-propen leichter abtrennen läßt als überschüssiges Sulfurylchlorid. Für die Reaktion selbst ist ein Überschuß an Sulfurylchlorid nicht nachteilig.

Die Beispiele dienen der Verdautlichung des erfindungsgemäßen Verfahrens.

**Beispiel 1**

In einer Rührapparatur mit Rückflußkühler und Tropftrichter, in die eine Quecksilber-Hochdrucktauchlampe eintaucht, werden 87 g stabilisatorfreies (Stabilisatorgehalt unter 1 ppm) 1-Chlor-2-methyl-propen vorgelegt, auf 45° C erwärmt und innerhalb von 2 Min. 108 g $SO_2Cl_2$ unter Thermostatisierung des Rührkolbens zudosiert.

Der Versuch wird einmal ohne und einmal mit Einwirkung von Licht, im übrigen in gleicher Weise durchgeführt. Das bei der Reaktion freiwerdende gasförmige $SO_2$ und gegebenenfalls etwas HCl wird über den Kühler abgezogen. Die Reaktion wird jeweils abgebrochen, wenn die $SO_2$-Entwicklung aufhört.

Danach wird das Rohprodukt mit Wasser gewaschen, über $K_2CO_3$ getrocknet und zur Ermittlung der Produktzusammensetzung einer gaschromatografischen Analyse unterworfen.

Die Versuchsergebnisse zeigt Tabelle 1.

**Tabelle 1**

Umsetzung von stabilisatorfreiem 1-Chlor-2-methyl-propen mit Sulfurylchlorid mit und ohne Einwirkung von UV-Licht

| UV-Licht | Reaktions-temperatur °C | Reaktions-zeit min | Produkt-menge g | Produktzusammensetzung, % *) 3,3-Dichlor-2-methyl-propen | 1,3-Dichlor-2-methyl-propen | 1,1,2-Trichlor-2-methyl-propan |
|---|---|---|---|---|---|---|
| ohne | 45 bis 50 | 300 | 138 | 12,5 | 1,5 | 82,0 |
| mit (erfin dungsgemäß | 45 bis 50 | 150 | 145 | 0,2 | 0,5 | 99,0 |

*) Rest auf 100 % nicht näher identifiziert

**Beispiel 2**

In einer Rührapparatur mit Rückflußkühler und Tropftrichter werden 90,6 g 1-Chlor-2-methyl-propen, frei von stickstoffhaltigen Stabilisatoren, vorgelegt, auf 45°C erwärmt, 100 ppm Isobutyraldehyd bzw. 1 000 ppm Acrolein zugegeben und innerhalb von 60 min 108 g $SO_2Cl_2$ unter Themostatisierung des Rührkolbens zudosiert.

Das bei der Reaktion freiwerdende und ausgasende $SO_2$ und gegebenenfalls etwas HCl wird über den Kühler abgezogen. Die Reaktion ist beendet, wenn die $SO_2$-Entwicklung aufhört.

Danach wird das Rohprodukt mit Wasser gewaschen, über $K_2CO_3$ entsäuert und getrocknet und zur Ermittlung der Produktzusammensetzung einer gaschromatografischen Analyse unterworfen.

Die Versuchsergebnisse zeigt Tabelle 2.

**Tabelle 2**

Umsetzung von stabilisatorfreiem 1-Chlor-2-methyl-propen mit Sulfurylchlorid in Gegenwart von Aldehyden

| Aldehyd | Reaktions-temperatur | Reaktionszeit, incl. Zudosier-zeit für $So_3Cl_2$ Gesamt-zeit | Zudosier-zeit | Rohprodukt-menge | Reinproduktzusammensetzung, % +) 3,3-Dichlor-2-methyl-propen | 1,3-Dichlor-2-methyl-propen | 1,1,2-Trichlor-2-methyl-propan |
|---|---|---|---|---|---|---|---|
| ppm | °C | min | min | g | | | |
| Isobutanal | | | | | | | |
| 100 | 45 - 50 | 240 | 60 | 144 | 1,5 | 1,0 | 96,0 |
| Acrolein | | | | | | | |
| 1 000 | 45 - 50 | 240 | 60 | 142 | 2,5 | 1,5 | 94,0 |

+) Rest auf 100 % nicht näher identifiert

**Patentansprüche**

1. Verfahren zur Herstellung von 1,1,2-Trichlor-2-methyl-propan,
dadurch gekennzeichnet,
daß man 1-Chlor-2-methyl-propen mit Sulfurylchlorid unter Einwirkung von Licht, insbesondere von UV-Licht, und/oder in Gegenwart von Aldehyden als Katalysatoren umsetzt.

2. Verfahren nach Anspruch 1,
dadurch gekennzeichnet,
daß man als Aldehyde gesättigte oder ungesättigte Aldehyde in einer Konzentration von 10 bis 10 000 ppm einsetzt.

3. Verfahren nach Anspruch 1 und 2,
dadurch gekennzeichnet,
daß man die Umsetzung unter Einwirkung von Licht und in Gegenwart von Aldehyden durchführt.

4. Verfahren nach Anspruch 1 bis 3,
dadurch gekennzeichnet,
daß man die Reaktion in Flüssigphase durchführt.

5. Verfahren nach Anspruch 1 bis 4,
dadurch gekennzeichnet,
daß man die Reaktion bei 30 bis 65° C unter Normaldruck durchführt.

6. Verfahren nach Anspruch 1 bis 5,
dadurch gekennzeichnet,
daß man Sulfurylchlorid, bezogen auf 1-Chlor-2-methyl-propen, im stöchiometrischen Unterschuß einsetzt.

**Claims**

1. A process for the production of 1,1,2-trichloro-2-methylpropane, characterised in that 1-chloro-2-methyl-propene is reacted with sulphuryl chloride under the action of light, especially UV light, and/or in the presence of an aldehyde as catalyst.

2. A process according to claim 1, characterised in that a saturated or unsaturated aldehyde is used as the aldehyde at a concentration of 10 to 10,000 ppm.

3. A process according to claim 1 or 2, characterised in that the reaction is carried out under the action of light and in the presence of an aldehyde.

4. A process according to any of claims 1 to 3, characterised in that the reaction is carried out in the liquid phase.

5. A process according to any of claims 1 to 4, characterised in that the reaction is carried out at 30 to 65° C under standard pressure.

6. A process according to any of claims 1 to 5, characterised in that sulphuryl chloride is used in a stoichiometrically deficient amount relative to 1-chloro-2-methylpropene.

**Revendications**

1. Procédé pour la préparation de 1,1,2-trichloro-2-méthyl-propane,
caractérisé par le fait que l'on fait réagir du 1-chloro-2-méthyl-propène sur du chlorure de sulfuryle en faisant agir de la lumière, en particulier de la lumière ultra-violette, et/ou en présence d'aldéhydes en tant que catalyseurs.

2. Procédé selon la revendication 1, caractérisé par le fait que l'on utilise comme aldéhydes, des aldéhydes saturés ou non saturés, dans une concentration de 10 à 10 000 ppm.

3. Procédé selon les revendications 1 et 2, caractérisé par le fait que l'on effectue la réaction sous l'action de la lumière et en présence d'aldéhydes.

4. Procédé selon les revendications 1 à 3, caractérisé par le fait que l'on effectue la réaction en phase liquide.

5. Procédé selon les revendications 1 à 4, caractérisé par le fait que l'on effectue la réaction à une température de 30 à 65°C sous la pression normale.

6. Procédé selon les revendications 1 à 5, caractérisé par le fait que l'on utilise le chlorure de sulfuryle dans une quantité stoechiométriquement insuffisante par rapport au 1-chloro-2-méthyl-propène.